# EUROPEAN PATENT SPECIFICATION

(11) **EP 2 759 294 B1**
(45) Date of publication and mention of the grant of the patent: **01.11.2017**
(21) Application number: 12833553.6
(22) Date of filing: 21.09.2012
(51) Int. Cl.: A61K 9/70, A61K 31/4468, A61P 17/00, A61K 47/10, A61K 47/16, A61K 47/22, A61K 31/4535, A61K 47/14

(54) **Formulation for percutaneous administration containing Fentanyl and analague thereof**
PERKUTAN ABSORBIERBARES PRÄPARAT MIT FENTANYL UND EINEM HOMOLOG DAVON
PRÉPARATION ABSORBABLE PAR VOIE PERCUTANÉE CONTENANT DU FENTANYL ET UN HOMOLOGUE DE CELUI-CI

(30) Priority: 22.09.2011 KR 20110095787
(43) Date of publication of application: 30.07.2014
(73) Proprietor: Samyang Biopharmaceuticals Corporation, Seoul 110-725 (KR)
(72) Inventor: YU, Hyun-Suk, Gunpo-si Gyeonggi-do 435-745 (KR); LEE, Young-Moo, Seoul 138-790 (KR); KIM, Hyun-Woo, Yongin-si Gyeonggi-do 448-110 (KR); KIM, Hee-Sook, Asan-si Chungnam 336-722 (KR)
(74) Representative: Krauns, Christian
(86) International application number: PCT/KR2012/007599
(87) International publication number: WO 2013/042989

(56) References cited:
- KR-A- 20010 036 685
- KR-A- 20030 021 418
- KR-A- 20050 046 350
- KR-A- 20060 049 598
- US-A- 6 139 866
- US-A1- 2004 234 584
- US-A1- 2005 095 279
- US-A1- 2009 004 257

## Description

### FIELD OF THE INVENTION

The present invention provides a transdermal preparation for administering fentanyl or analogue thereof, as defined by the claims, and more specifically, the transdermal preparation having an improved permeation efficiency of the formulation by lowering the drug dosage in the formulation, the increased safety of drug remained in the formulation after using, and the use convenience by decreasing the formulation area.

### BACKGROUND ART

The fentanyl or an analogue thereof is a strong synthetic opioid pain reliever and has been approved by a general anesthetic drug. The fentanyl or analogue thereof has a relatively limited guidance of medical treatment. The term, 'efficacy presence' means that a relatively-low blood concentration of the drug can provide a sufficient efficacy. The phrase 'a limited guidance of medical treatment' means that only limited concentration ranges of drug can achieve the efficacy. However, the drug cannot have a sufficient efficacy at lower concentration than the proper range, and can cause the side-effect at a higher concentration than the proper range. Therefore, the administration of formulation containing the fentanyl or an analogue thereof should be initiated at a dosage which is expected to guarantee the patient's safety, and then be increased gradually to the range of the proper painkilling. Because the drug tolerance and the pain may become higher over time, the drug should treat pain in combination with other pain reliever.

The fentanyl or an analogue thereof as an opioid pain reliever can be administered by various administration preparations, for examples local administration preparations such as ointment, cream, parenteral injection and lollipop; and the preparation for percutaneous administration. In the preparation for percutaneous administration, the drug is typically transferred from the contacting side of the formulation containing the adhesive layer of pressure-sensitive adhesive and the drug.

An example of the preparation for percutaneous administration describes a multilayer film including a drug releasing controlling layer located between the drug reservoir and the skin contacting adhesive layer, the drug releasing controlling layer reduces the change in the effect of skin permeation by decreasing the drug releasing rate in vitro from the formulation. However, the formulation is suitable for the drug with a high efficacy, but needs a larger area of formulation to achieve the same administration rate, thereby not being good for use convenience.

Depending on the solubility of drug in the preparation for percutaneous administration, the formulations are divided into an unsaturated patch containing a drug dissolved completely in the drug reservoir and a supersaturated patch containing the undissolved drug at a more amount than the saturation amount. From the initial time of patch attachment, the saturation degree of drug in the unsaturated patch is reduced continuously and thus, the drug administration rate can be decreased continuously during usage. In the supersaturated patch, the excessive amount of drug can maintain the saturation degree in the drug reservoir. The supersaturated patch can achieve the maximum drug transfer rate, so long as the excessive amount of drug is in the drug reservoir. The supersaturated patch is advantageous, in case that the drug must be administrated at relatively consistent rate. However, undissolved drug and other additives in the formulation cause the stability problem in the storage and usage.

For example, the commercially available product of the supersaturated patch is DURAGESIC(®) including a backing layer of impermeable film and a drug reservoir layer. In the drug reservoir, the fentanyl is dissolved in a mixture of water and ethanol at the supersaturated state. This formulation includes a permeable membrane between the drug reservoir and an adhesive layer to attach it onto the skin. In case that the drug leaks from the patch, filling materials containing the drug contact with a large area of skin, thereby causing the drug to be administered at excessively high rate. When the fentanyl is administered at an excessively large amount, it may cause the acute hypoventilation. Various lethal cases and similar cases have been reported [Clinical Pharmacokinet. 2000, 38(1), 59-89].

DURAGESIC D-trans (®) was provided as the unsaturated patch for solving the problems of supersaturated patch. As the related patent documents, KR 2003-0082995A discloses a method of using polyacrylate adhesive including a hydroxyl group (Durotak2287, 4287). US 4,588,580 discloses a matrix-type transdermal system in which fentanyl is filled in the silicon or polyisobutylene adhesive. In these documents, the skin permeation rate of fentanyl is very low, when the polyacrylate adhesive having a carboxyl group is used. In case of silicone-based adhesive, fentanyl cannot be contained at a large amount in the formulation, due to a low solubility of fentanyl to the silicone-based adhesive, and can permeate the skin a very short time due to a high diffusivity in the silicon-based adhesive, thereby making the formulation be used difficultly as a long-term efficacy drug. In addition, when the rubber-based adhesive such as polyisobutylene is used in the drug adhesive layer, the change in the material property is serious due to the aging and the extraction of drug crystallization is rapid. It cannot be suitable for the drug required for a long-term efficacy drug, because it does not have a sufficient adhesiveness.

Another example of trandermal patch, Matrifen patch uses an amine-resistant silicone adhesive to increase the solubility of fentanyl, in combination with other additives. Matrifen patch contains the ethylene vinylacetate (EVA) film in order to prevent the fentanyl permeating the skin at a short time and thus has disadvantages in the complicated structure and a high production cost. US 5,186,939 discloses a matrix-type transdermal preparation containing a silicone-based adhesive and a propylene glycol monolaruate as a permeation enhancer of drug. However, the preparation releases most drugs in 24 hours and thus, is not suitable for the drug with the long-term effect.

In order to improve the properties of monolithic unsaturated patch such as DURAGESIC D-trans (®), the present invention provide an equal or higher permeation efficiency of the formulation by using a specific skin permeation enhancer of drug, although the drug dosage is reduced in the formulation. The present invention also solves a safety problem of drug remained in the formulation after using, and provides the use convenience by reducing the attaching area of the formulation.

US 2005/0095279 A1 relates to a transdermal analgesic system having reduced potential for abuse, wherein the system provides for the controlled release of the antagonist at a rate sufficient to provide an abuse limiting release rate ratio of the antagonist to the analgesic when the dosage form is subject to abuse.

### SUMMARY OF THE INVENTION

An embodiment of the present invention provides a transdermal preparation having improved permeation efficiency by using a specific permeation enhancer of as defined by the claims, wherein the preparation can achieve the equivalent or higher permeation rate in spite of a lower drug dosage than that of the conventional art.

The preparation can solve the safety problem caused by the remaining drug after usage and provide the use convenience by decreasing the attaching area of formulation.

### DETAILED DESCRIPTION

Hereinafter, the present invention will be described in more detail.

An embodiment of the present invention is to provide a transdermal preparation as defined by the claims for administering fentanyl or an analogue thereof, comprising a backing layer (10), a barrier layer (20), a drug adhesive layer (30) and a release layer (40) which are stacked sequentially, wherein the drug adhesive layer comprises (a) a drug selected from the group consisting of fentanyl, an analogue and a pharmaceutically-acceptable salt thereof, (b) a permeation enhancer of the drug, and (c) one or more polyacrylate adhesives selected from the group consisting of a non-functional polyacrylate adhesive and a carboxyl-containing polyacrylate adhesive.

Another embodiment of the present invention is to provide a transdermal preparation for administering fentanyl, or an analogue or a pharmaceutically acceptable salt thereof, having 1.05 to 10.08 mg of the drug content per unit dosage(patch), 0.4 to 0.6 mg/cm² of the drug content per unit area of the formulation, 3.0 to 6.5 *µ*g/hr/cm² of the drug permeation rate and 50 to 95 wt% of drug releasing amount per unit dosage to the initial loading amount of drug as solid.

The formulation of the present invention increases the permeation efficiency of the drug, even with the low drug amount per unit dosage, solves a safety problem of drug remained in the formulation after using the formulation, and provides the use convenience by reducing the area of the formulation.

The drug permeation amount can be measured by in vitro drug releasing test. The drug permeation rate can be measured by using Franz diffusion cell system under the similar condition to the skin surface.

In accordance with an embodiment of the present invention, the drug being suitable for the drug adhesive layer (30) is a fentanyl (or Fentanil) or an analogue thereof, for example, at least one selected from the group consisting of alfentanil, sufentanyl, remifentanyl, 3-methylfentanyl, carfentanyl, lofentanyl, trefentenyl and a pharmaceutically-acceptable salt thereof.

The total amount of drug contained in the drug adhesive layer (30) can be determined depending on the concentration and the formulation area being suitable for achieving the significant effect, under the condition including the drug and other additives. The total amount of drug is 1 to 20 wt% or preferably 4 to 10 wt% based on the solid content of drug adhesive layer (30). When the drug amount is higher than 20 wt%, the drug can be extracted as a crystal, because of the high drug concentration of the drug adhesive layer (30). When the drug amount is less than 1 wt%, the skin permeation rate is excessively low and thus, cannot achieve the significant effect because of a low drug concentration of drug adhesive layer.

The dry thickness of drug adhesive layer (30) is 10 to 200 *µ*m, or preferably 30 to 120 *µ*m.

The skin permeation enhancer of drug in an embodiment is used for increasing the skin permeation of drug through the cornified layer into the body. The examples of the skin permeation enhancers include a surfactant capable of increasing the skin permeation, solubility enhancing agent, tackifying resin, stabilizer, plasticizer, antioxidant, flavoring agent, pH adjusting agent, excipient and anti-irritating agent, but not limited thereto and the functional name of material.

The examples of surfactant being useful as a skin permeation enhancer include non-ionic surfactant, anionic surfactant, cationic surfactant, amphoteric surfactant and a mixture thereof

The non-ionic surfactant being useful as a skin permeation enhancer means a surfactant which is not dissociated into ion and is not charged, and includes the compounds prepared by the condensation reaction of hydrophobic organic compounds such as aliphatic hydrocarbon and alkyl aromatic compound, with alkylene oxide group (hydrophilic). For examples, the non-ionic surfactant includes a condensation product of alkyl phenol with polyethylene oxide, polypropylene oxide or polybutylene oxide; fatty acid amide surfactant; polyhydroxy fatty acid amide surfactant; amine oxide surfactant; alkyl ethoxylate surfactant, alkanoyl glucose amide surfactant, alkanol amide surfactant, alkylpolyglycoside and a condensation product of aliphatic alcohol and about 1 to 25 moles of ethylene oxide. Preferably, the non-ionic surfactant includes fatty acid alkanolamide compounds of C₅₋₂₂ fatty acids such as Cocamide DEA, Cocamide MEA, Cocamide MIPA, PEG-5 Cocamide MEA, Lauramide DEA, and Lauramide MEA; fatty acid alkylamine oxide compounds of C₈₋₁₈ fatty acids such as lauramine oxide, cocamine oxide, cocamidopropylamine oxide, and lauramidopropylamine oxide; fatty acid ester compounds of C₁₈₋₆₄ sorbitan such as sorbitan laurate, sorbitan distearate, PEG-80 sorbitan laurate, polysorbate-20, polysorbate-60 and polysorbate-80; fatty acids or fatty acid esters such as lauric acid, isostearic acid and PEG-150 distearate; fatty acid alcohols or ethoxylated fatty acid alcohols such as lauryl alcohol, laureth-4, laureth-7, laureth-9, laureth-40, trideceth alcohol, C11-15 Pareth-9, C12-13 Pareth-3, C14-15 Pareth-11 and the like.

The examples of anionic surfactants include alkyl and alkyl ether sulfate, sulfated monoglyceride, sulfonated olefin, alkyl aryl sulfonate, primary or secondary alkane sulfonate, alkyl sulfosuccinate, acyl taurate, acylisethionate, alkyl glycerylether sulfonate, sulfonated methyl ester, sulfonated fatty acid, alkyl phosphate, acyl glutamate, acyl sarcosinate, alkyl sulfoacetate, acylated peptide, alkyl ether carboxylate, acyl lactylate, anionic fluorosurfactant and a mixture thereof.

The examples of the amphoteric surfactants include the derivatives of aliphatic secondary or tertiary amine where the aliphatic radical may be linear or branched, one of the aliphatic radicals may include C₈₋₁₈ carbon atoms and the other of the aliphatic radicals include water-soluble groups such as carboxyl, sulfonate, sulfate, phosphate or phosphonate which are ionizable. Specifically, the amphoteric surfactants include alkyl iminopropionate, alkyl iminodipropionate, and alkyl amphopropyl sulfonate such as cocoamphoacetate, cocoamphopropionate, cocoamphodiacetate, lauroamphoacetate, lauroamphodiacetate, lauroamphodipropionate, lauroamphodiacetate, cocoamphopropyl sulfonate, carpro amphodiacetate, caproamphoacetate, caproamphodipropionate, or stearoamphoacetate; alkaline metal salt, alkaline earth metal salt, ammonium salt or substituted ammonium salt and alkylbetaine of alkyl amphocarboxyglycinate and alkyl amphocarboxypropionate, alkyl amphodipropionate, alkyl amphodiacetate, alkyl amphoglycinate, or alkyl amphopropionate, such as cocodimethyl carboxymethyl betaine, lauryldimethylcarboxymethyl betaine, lauryldimethyl alpha-carboxy-ethyl betaine, cetyldimethylcarboxymethyl betaine, lauryl bis-(2-hydroxy-ethyl)carboxymethyl betaine, stearyl bis-(2-hydroxypropyl)carboxymethyl betaine, oleyldimethyl gamma-carboxypropyl betaine and lauryl bis(2-hydroxypropyl) alpha-carboxyethyl betaine, amidopropyl betaine and alkyl sultaine such as cocodimethylsulfopropyl betaine, stearyldimethyl sulfopropyl betaine, lauryl dimethyl sulfoethyl betaine, lauryl bis-(2-hydroxy-ethyl)sulfopropyl betaine, and alkyl amidopropyl hydroxyl sultaine.

For examples, the solubility enhancing agent being applicable to the skin permeation enhancer are ethanol, methanol, methylene chloride, toluene, hexane, heptanes, 2-propanol, 1-propanol, 1-butanol, 2-butanol, 1-pentanol, ethylacetate, pyrrolidone derivatives such as N-dodecyl-2-pyrrolidone(lauryl pyrrolidone), N-methylpyrrolidone, N-methyl-2-pyrrolidone, 1,5-dimethyl-2-pyrrolidone, 1-ethyl-2-pyrrolidone, 1-hexyl-2-pyrrolidone, 1-(2-hydroxyethyl)pyrrolidone, 1-lauryl-4-methyloxycarbonyl-2-pyrrolidone and N-caprylyl-2-pyrrolidone, triacetin, Transcutol(diethyleneglycolmonoether), levulinic acid and the like. Because the solvent contained in the adhesive needs to be removed by drying at a high temperature in the process of preparing a transdermal formulation of the present invention, the solvent must have the boiling point or volatilizing point being higher than the drying temperature of the transdermal formulation and have no skin-irritation. Thus, preferred examples of the solubility enhancing agent are N-dodecyl-2-pyrrolidone (lauryl pyrrolidone), N-methyl pyrrolidone and N-methyl-2-pyrrolidone. The skin permeation enhancer is at least one selected from the group consisting of lauramine oxide and N-dodecyl-2-pyrrolidone (lauryl pyrrolidone).

The skin permeation enhancer may be contained at 0.1 to 50 wt% or preferably 0.1 to 30 wt% based on the solid content of drug adhesive layer (30).

The (c) polyacrylate adhesive being useful for drug adhesive layer (30) of the present invention is selected from the group consisting of a non-functional polyacrylate adhesive and a carboxyl-containing polyacrylate adhesive, depending on the kinds of monomer. The polyacrylate adhesive shows various adhesive property and physiochemical property depending on the polymerization degree, and is used alone or in combination with other polyacrylate adhesive.

The examples of the monomer being used for polymerizing the polyacrylate adhesive are C₂₋₂₀ monomers such as acrylic acid, alkyl acrylate and alkyl methacrylate where the alkyl group is substituted or unsubstituted C₁₋₁₂ alkyl. Specifically, the examples of the alkyl acrylate or alkyl methacrylate are at least one selected from the group consisting of 2-ethylhexyl acrylate, hexyl acrylate, butylacrylate, ethylacrylate, isooctylacrylate, vinylacetate, methylacrylate, methylmethacrylate, ethylmethacrylate, acrylonitrile, hydroxyethylacrylate, octylacrylate, and t-octylacrylamide. The polyacrylate adhesive can be prepared by polymerizing the monomer in the presence of a polymerization initiator, and the polymerization method and condition are applied by those of the general method used for polymerizing the acrylate polymer.

The (meth)acrylic acid or (meth)acrylate can be polymerized to produce the nonfunctional polyacrylate adhesive, and one or two kinds of carboxyl-containing monomers can be polymerized to produce carboxyl-containing polyacrylate adhesive.

The examples of carboxyl-containing monomers are acrylic acid, methacrylic acid, crotonic acid, itaconic acid, fumaric acid, and maleic acid, and one or two monomers selected from them can be polymerized to produce the polyacrylate adhesive in drug adhesive layer.

The carboxyl-containing polyacrylate adhesive in accordance with the present invention is a polymer polymerized from at least one monomer selected from the group consisting of acrylic acid, methacrylic acid, crotonic acid, itaconic acid, fumaric acid, and maleic acid; or a copolymer polymerized from at least a monomer selected from the group consisting of acrylic acid, methacrylic acid, crotonic acid, itaconic acid, fumaric acid, and maleic acid, and at least a monomer selected from the group consisting of acrylic acid, 2-ethylhexyl acrylate, hexyl acrylate, butyl acrylate, ethyl acrylate, isooctylacrylate, vinyl acetate, methyl acrylate, methylmethacrylate, ethylmethacrylate, acrylonitrile, hydroxyethylacrylate, octylacrylate, and t-octylacrylamide. The carboxyl-containing polyacrylate adhesive may be acrylate vinylacetate copolymer, but is not limited thereto.

In an embodiment of the present invention, by using the proper skin permeation enhancer, the carboxyl-containing polyacrylate adhesive, which has not been used for the formulation of the fentanyl or an analogue thereof due to a low skin permeation rate, can be used with a sufficient permeation rate.

The polyacrylate adhesive being applicable for the present invention may have a weight-average molecular weight of 800,000 to 1,000,000 and the polymerization degree of 85 to 95, but not limited thereto.

The amount of polyacrylate adhesive in the drug adhesive layer (30) may be 55 to 99 wt%, based on the solid content of drug adhesive layer (30). The nonfunctional polyacrylate adhesive can be used alone at an amount of 80 to 99 wt%, and the carboxyl-containing polyacrylate adhesive can be used alone at an amount of 55 to 80 wt%. When the mixture of nonfunctional polyacrylate adhesive and carboxyl-containing polyacrylate adhesive is used, the mixture can be used at an amount of 80 to 99 wt% based on the solid content of drug adhesive layer (30) where the mixing weight ratio of the nonfunctional polyacrylate adhesive and the carboxyl-containing polyacrylate adhesive is 95 : 5 to 55 : 45 or preferably 90 : 10 to 60 : 40.

The barrier layer of the present invention can improve the skin permeation of drug and adhesiveness, and prevent the drug crystallization, and includes a rubber-based adhesive. The rubber-based adhesive being applicable to the barrier layer are natural or synthetic rubber compounds, for examples at least one selected from the group consisting of polyisobutylene, polyisoprene, polybutadiene, polybutene and a mixture thereof.

Besides the rubber-based adhesive, the barrier layer can include optionally at least one selected from the group consisting of a tackifying resin and a plasticizer, in order to provide or improve the adhesive property.

The examples of tackifying resins include C3-12 rosin and its derivative, C3-12 saturated aliphatic cyclic hydrocarbon resin, C3-12 aliphatic hydrocarbon resin, terpene resin, maleic acid resin and the like, the rosin derivative includes glycerol ester of rosin, hydrogenated rosin, glycerol ester of hydrogenated rosin, pentaerythritol ester of rosin and the like.

The examples of plasticizers include petroleum oil (e.g., paraffin processed oil, naphthalenic processed oil, aromatic processed oil, etc.), squalane, plant oil (e.g., olive oil, camellia oil, castor oil, tall oil, peanut oil, etc.), silicone oil, dibasic acid ester (e.g., dibutyl phthalate, dioctyl phthalate, etc.), liquid rubber (e.g., polybutene, liquid isoprene rubber, etc.), liquid fatty acid ester (e.g., isopropyl myristate, hexyl laurate, diethyl sebacate, diisopropyl sebacate, etc.), diethylene glycol, polyethylene glycol, glycol salicylate, propylene glycol, dipropylene glycol, triacetin, and the like.

In the specific examples of barrier layer, polyisobutylene such as Oppanol B100(BASF, Germany), and HIMOL 5H(Japan Petrochemical LTD, Japan) may be used as the rubber-based adhesives, and a product made from aliphatic hydrocarbon resin Escorez 1401(Exxonmobile, U.S.A.) and a suitable solvent is used as the tackifier resin.

The barrier layer of the present invention may have a dry thickness of 10 to 60 *µ*m, or preferably 20 to 40 *µ*m. When the dry thickness is less than 10 *µ*m, the prevention of drug crystallization and increased adhesiveness cannot be achieved. When the dry thickness is higher than 60 *µ*m, more improvement in the skin permeation cannot be obtained and feeling of irritation in formulation use may increase due to the high thickness.

The backing layer (10) of the present invention gives the mechanical property of the transdermal formulation and functions as a supporter, and can be formed by using the material being capable of attaching to the adhesive layer and maintaining the shape with no specific limitation on the material.

The material examples being applicable to the backing layer include at least one selected from the group consisting of polyester, polyolefin, polyurethane, polyvinylacetate, polyvinylidene chloride, polyethylene, ethylene vinyl acetate, polyethylene terephthalate, polybutylene terephthalate, and polyamide. The material shapes of the backing layer are sheet, film, fiber, fabric, knitted material and nonwoven fabric. The materials of backing layer can be the permeable or impermeable material including laminates which is prepared by using at least two kinds of the polymers with the porous film. As an example, a laminated film with a thickness of 15 to 50 *µ*m which is formed from ethylenevinyl acetate film and polyester film can be used for the backing layer, but not limited thereto.

The release layer of the present invention protects the drug adhesive layer (30) and is located under the drug adhesive layer, until the transdermal formulation is used. Any material satisfying the function of release layer can be used without limitation on the material or shape.

The examples of materials include at least an impermeable material selected from the group consisting of polyester, polyvinylacetate, polyvinylidene chloride, polypropylene, polystyrene, polycarbonate, ethylenevinyl acetate, polyethylene, polyethylene terephthalate, polybutylene terephthalate, aluminum sheet, paper and a mixture thereof. For example, the materials of release layer can be the polyester film which is treated with silicone or fluorine and has a thickness of 50 to 120 *µ*m.

As described above, the present invention provides a transdermal preparation, comprising a backing layer (10), a barrier layer (20), a drug adhesive layer (30) and a release layer (40) which are stacked sequentially, wherein the drug adhesive layer comprising (a) a drug selected from the group consisting of fentanyl, an analogue and a pharmaceutically-acceptable salt thereof, (b) a skin permeation enhancer of the drug, and (c) a polyacrylate adhesive. The transdermal preparation has an improved permeation efficiency of the formulation by achieving an equivalent effect in spite of the decreased drug dosage, the increased safety of drug remained in the formulation after using the formulation and the attaching convenience by decreasing the formulation area.

### BRIEF DESCRIPTION OF THE DRAWINGS

FIG. 1 is a cross-sectional diagram of the transdermal preparation of an embodiment of the present invention showing the stacked layers.
FIG. 2 is a cross-sectional diagram of the transdermal preparation of a conventional technology showing the stacked layers.
FIG. 3 is a graph showing the result of in vitro test for the skin permeation of the transdermal preparation of the Example and the Comparative Examples.
FIG. 4 is a graph showing the result of in vitro test for the skin permeation of the transdermal preparation of the Examples and the Comparative Examples.
FIG. 5 is a graph showing the result of in vitro test for the skin permeation of the transdermal preparation of the Examples and the Comparative Examples.
FIG. 6 is a graph showing the result of in vitro test for the skin permeation of the transdermal preparation of the Examples and the Comparative Examples.

### [Example]

Hereinafter, the present invention will be described in detail with reference to examples and experimental examples, which are given only for illustrative purposes and not intended to limit the scope of the present invention.

When being defined herein particularly otherwise, the nonfunctional polyacrylate adhesive used in the Examples and Comparative Examples was acrylic acid ester copolymer TRM-05NF(Soken Chemical Co.,Ltd., Japan) having a molecular weight of 850,000 to 950,000 and a polymerization degree of 85 to 95. The acrylate/vinylacetate copolymer DURO-TAK 387-2052 (Henkel, U.S.A.) was used as a carboxyl-containing polyacrylate adhesive. BioPSA 4302 (Dowcorning, U.S.A.) dissolved in ethyl acetate as a solvent was used as a silicone-based adhesive. Polyisobutylene-based adhesives, Oppanol B100(BASF, Germany) and HIMOL 5H(Japan Petrochemical LTD, Japan), were used as a rubber-based adhesive. CW-901R(Chembase Inc, Korea) including aliphatic hydrocarbon resin Escorez 1401(Exxonmobile, U.S.A.) was used as a tackifier resin.

### <Example 1>

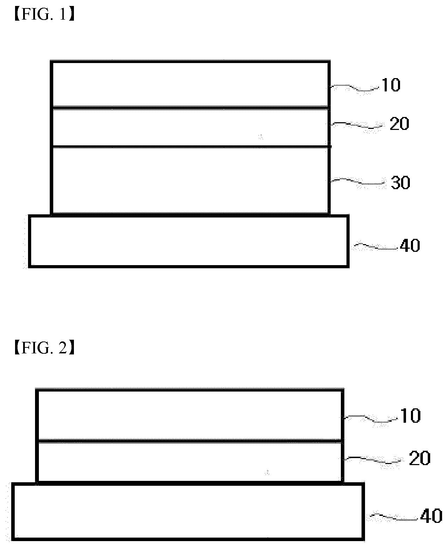

To prepare the trandermal preparation in FIG. 1, the barrier layer (20) was made by coating an rubber-based adhesive solution (CW-901R, Chembase Inc, Korea) containing polyisobutylene and aliphatic hydrocarbon resin on the silicon-coated polyester film to be 30 *µ*m of the dried film thickness of rubber-based adhesive solution, drying at 90 °C for 10 minutes, covering with a laminated film of ethylene vinyl acetate film and a polyester film as a support film, and then giving the pressure on it.

To prepare the drug adhesive layer, a sufficient amount of fentayl was added to be 10.0 wt% of fentanyl as solid, into as a nonfunctional polyacrylate adhesive solution acrylic acid ester copolymer (TRM-05NF, Soken Chemical Co. Ltd., Japan), agitated sufficiently to dissolve the drug, and then was coated on the silicon-coated polyester film to be 0.4 mg/cm² of fentanyl and dried at 90 °C for 10 minutes.

The polyester film was removed from the laminate of backing layer and barrier layer, and the laminate was closely positioned with the drug adhesive layer so as not to form air bubble, and was laminated with proper pressure to produce the trandermal patch.

### <Comparative Examples 1 to 3>

Comparative Example 1 was to prepare the trandermal preparation in FIG. 2 and included the same drug adhesive layer of Example 1, except that the barrier layer was not contained.

Comparative Example 2 was to prepare the trandermal preparation and included the same drug adhesive layer of Example 1, except that the barrier layer contained a silicone-based adhesive BioPSA 4302 (Dowcorning, U.S.A.) instead of the rubber-based adhesive of Example 1.

Comparative Example 3 was to prepare the trandermal preparation and included the same drug adhesive layer of Example 1, except that the barrier layer contained carboxyl-containing polyacrylate adhesive DURO-TAK 387-2052 (Henkel, U.S.A.) as acrylate-vinyl acetate copolymer, instead of the rubber-based adhesive of Example 1.

### <Test Example 1>

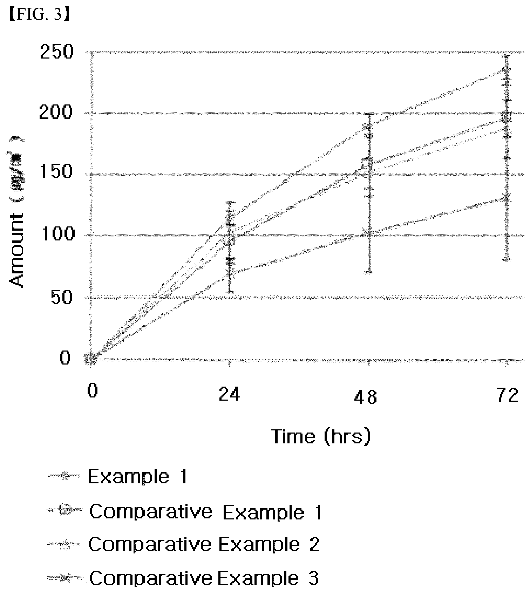

The transdermal preparations obtained by Example 1 and Comparative Examples 1 to 3 were tested for the skin permeation according to the following method. The test result was shown in Table 1 and FIG. 3.

### <Skin permeation test >

The preparations were added to Franz diffusion cell system and tested at 32±0.5°C for the skin permeation. Phosphate buffer solution (pH=7.4) was used as a release solution. The preparations prepared were attached onto Cadaver skin and were fixed to the central part of the Franz diffusion apparatus, and then the release solution was agitated at 600rpm. At certain time interval, each 100 *µℓ* of release solution was taken and the supplemented with the same amount of phosphate buffer. The drug in the taken solution was analyzed with HPLC under the following condition, and the drug amount was determined from Calibration Curve.

### < HPLC Analysis Condition of fentanyl>

Column: Capcell Pak C₁₈ UG120, 4.6 mm × 150 mm (Shiseido CO., LTD., Japan)
Mobile phase: 0.2% HClO₄ (pH 2.3 adjusted with NaOH : ACN = 65 : 35
UV: 210 nm
Flow rate: 1.0 mℓ/minute
Sample injection amount: 10 *µℓ*
[Mathematical Formula 1]
Concentration of test liquid (C, µg/ml) = (peak area of test liquid-intercept of calibration curve)/slope of calibration curve)
[Mathematical Formula 2]
Amount of released drug (A, µg/each patch)
= {((Cn x V) + (C1+C2+...+Cn) x 0.1)/As} x At
[Mathematical Formula 3]
Skin permeation rate (µg/hr/cm²) = (A/At) x (1/T)

The factors in Mathematical Formulae 1 to 3 are defined as follow:
n = number of collecting the test liquid,
V = volume of Franz diffusion cell (mℓ),
0.1 = volume of collected test liquid (mℓ),
As = test body area (cm²),
At = area of one patch (cm²), and
T = time of collecting the test liquid (hr).

**[Table 1] Composition and properties of the preparations**

| Classification | | Example 1 | Comparative Example 1 | Comparative Example 2 | Comparative Example 3 |
|---|---|---|---|---|---|
| drug adhesive layer (wt%) | fentanyl | 10 | 10 | 10 | 10 |
| | nonfunctional polyacrylate adhesive | 90 | 90 | 90 | 90 |
| | Total amount of drug adhesive layer | 100 | 100 | 100 | 100 |
| | drug adhesive layer thickness (*µ*m) | 40 | 40 | 40 | 40 |
| barrier layer (thickness *µ*m) | Rubber-based adhesive | 30 | - | - | - |
| | Silicone-based adhesive | - | - | 30 | - |
| | Carboxyl-containing polyacrylate adhesive | - | - | - | 30 |
| Area per patch (cm²) | | 20 | 20 | 20 | 20 |
| Drug usage amount (mg/patch) | | 8.4 | 8.4 | 8.4 | 8.4 |
| Drug amount per unit area (mg/cm²) | | 0.42 | 0.42 | 0.42 | 0.42 |
| Drug permeation amount (mg/patch) | | 4.73 | 3.39 | 3.76 | 2.63 |
| Skin permeation rate(*µ*g/hr/cm²) | | 3.28 | 2.73 | 2.61 | 1.83 |
| Drug release amount per patch (%) | | 56.31 | 46.79 | 44.76 | 31.31 |

As shown in Table 1 and FIG. 3, Example 1 using the rubber-based adhesive in barrier layer represented high skin permeation rate, compared to the preparation without the barrier layer in Comparative Example 1, and the preparations using the silicone-based adhesive or polyacrylate adhesive in Comparative Examples 2 or 3.

### <Example 2>

The transdermal preparation shown in FIG. 1 was prepared according to the same method of Example 1, except that the drug adhesive layer contained 92.83 wt% of acrylic acid ester copolymer TRM-05NF (Soken Chemical Co.,Ltd., Japan) as the nonfunctional polyacrylate adhesive, 6.67 wt% of fentanyl, and 0.5 wt% of lauramine oxide as a skin permeation enhancer, so as to include 0.42 mg/cm² of fentanyl as a dry weight.

### <Example 3>

The transdermal preparation was prepared according to the same method of Example 2, except that the drug adhesive layer contained 0.504 mg/cm² of fentanyl as a dry weight.

### <Example 4>

The transdermal preparation was prepared according to the same method of Example 2, except that the drug adhesive layer contained 0.504 mg/cm² of fentanyl as a dry weight and the skin permeation enhancer was 5.0 wt% of N-dodecyl-2-pyrrolidone (lauryl pyrrolidone).

### <Example 5>

The transdermal preparation shown in FIG. 1 was prepared according to the same method of Example 1, except that the drug adhesive layer contained 88.7 wt% of acrylic acid ester copolymer TRM-05NF (Soken Chemical Co.,Ltd., Japan) as the nonfunctional polyacrylate adhesive, 5.7 wt% of fentanyl, and a mixture of 0.5 wt% of lauramine oxide and 5.0 wt% of N-dodecyl-2-pyrrolidone (lauryl pyrrolidone) as a skin permeation enhancer, so as to include 0.504 mg/cm² of fentanyl as a dry weight.

### <Comparative Examples 4 to 5>

The transdermal preparation shown in FIG. 1 was prepared according to the same method of Example 1, except that the drug adhesive layer contained 80 wt% of acrylic acid ester copolymer TRM-05NF (Soken Chemical Co.,Ltd., Japan) as the nonfunctional polyacrylate adhesive and 10 wt% of fentanyl, and as the skin permeation enhancers, 10 wt% of Dipropylene glycol was used in Comparative Example 4 and 10 wt% of Glyceryl monolaurate was used in Comparative Example 5, so as to include 0.504 mg/cm² of fentanyl as a dry weight.

### <Example 6>

The transdermal preparation shown in FIG. 1 was prepared according to the same method of Example 1, except that the drug adhesive layer contained 67.0 wt% of acrylate/vinylacetate copoplymer DURO-TAK 387-2052 (Henkel, U.S.A.) as a carboxyl-containing polyacrylate adhesive and 8.0 wt% of fentanyl, and 25 wt% of lauramine oxide as a skin permeation enhancer, so as to include 0.4 mg/cm² of fentanyl as a dry weight.

### <Example 7>

The transdermal preparation shown in FIG. 1 was prepared according to the same method of Example 1, except that the drug adhesive layer contained 75.0 wt% of acrylic acid ester copoplymer TRM-05NF(Soken Chemical Co.,Ltd., Japan) as a nonfunctional polyacrylate adhesive, 8.33 wt% of acrylate/vinylacetate copolymer DURO-TAK 387-2052 (Henkel, U.S.A.) as a carboxyl-containing polyacrylate adhesive where the mixing weight ratio of the nonfunctional polyacrylate adhesive and the carboxyl-containing polyacrylate adhesive was 90 : 10 based on the solid content, 6.67 wt% of fentanyl and 10 wt% of lauramine oxide as a skin permeation enhancer, so as to include 0.42 mg/cm² of fentanyl as a dry weight.

### <Comparative Examples 6 to 8>

The preparation of Comparative Example 6 was prepared according to the same method of Example 5, except that the permeation enhancer of drug was not used.

The preparation of Comparative Example 7 was prepared according to the same method of Example 5, except that Glyceryl monolaurate was used as the skin permeation enhancer of drug.

The preparation of Comparative Example 8 was prepared according to the same method of Example 6, except that the permeation enhancer of drug was not used.

### <Test Example 2>

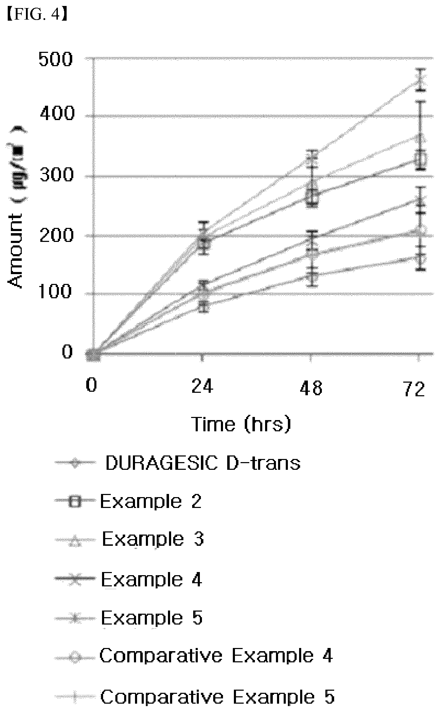

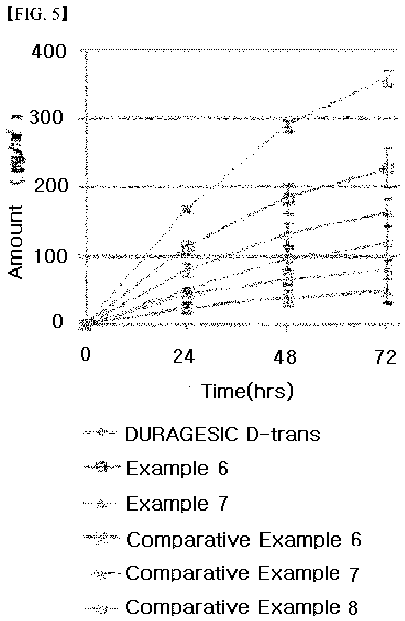

The transdermal preparations obtained in Examples 2 to 7 and Comparative Examples 4 to 8 were tested according to the same method of Test Example 1 and the test results of Examples 2 to 5 and Comparative Examples 4 to 5 were shown in FIG. 4, and the test results of Examples 6 to 7 and Comparative Examples 6 to 8 were shown in FIG. 5. DURAGESIC D-trans (®) was used as a control (100 *µ*g/hr, 16.8 mg/42 cm²), the skin permeation rate was 2.26 *µ*g/hr/cm² and the skin permeation amount of drug was 6.83 mg/patch.

**[Table 2] The compositions and physiochemical properties of Examples 2 to 5 and Comparative Examples 4 to 5**

| classification | | Example 2 | Example 3 | Example 4 | Example 5 | Comparative Example 4 | Comparative Example 5 |
|---|---|---|---|---|---|---|---|
| drug adhesive layer (wt%) | fentanyl | 6.67 | 6.67 | 6.67 | 5.7 | 10 | 10 |
| | nonfunctional polyacrylate adhesive | 92.83 | 92.83 | 88.33 | 88.7 | 80 | 80 |
| | Lauramine oxide | 0.5 | 0.5 | - | 0.5 | - | - |
| | Lauryl pyrrolidone | - | - | 5.0 | 5.0 | - | - |
| | Dipropylene glycol | - | - | - | - | 10 | - |
| | Glyceryl monolaurate | - | - | - | - | - | 10 |
| | Sum | 100 | 100 | 100 | 100 | 100 | 100 |
| | drug adhesive layer thickness (*µ*m) | 63.0 | 75.6 | 75.6 | 88.4 | 50.4 | 50.4 |
| barrier layer thickenss *µ*m) | Rubber-based adhesive | 30 | 30 | 30 | 30 | 30 | 30 |
| Area per patch (cm²) | | 20 | 20 | 20 | 20 | 20 | 20 |
| Drug usage amount (mg/patch) | | 8.4 | 10.08 | 10.08 | 10.08 | 10.08 | 10.08 |
| Drug amount per unit area (mg/cm²) | | 0.42 | 0.504 | 0.504 | 0.504 | 0.504 | 0.504 |
| Drug permeation amount (mg/patch) | | 6.59 | 7.38 | 5.22 | 9.27 | 4.2 | 4.12 |
| Skin permeation rate (*µ*g/hr/cm²) | | 4.57 | 5.13 | 3.63 | 6.44 | 2.92 | 2.86 |
| Drug release amount per patch (%) | | 78.45 | 73.21 | 51.8 | 91.96 | 41.67 | 40.87 |

**[Table 3] The compositions and physiochemical properties of Examples 6 to 7 and Comparative Examples 6 to 8**

| classification | | Example 6 | Example 7 | Comp. Example 6 | Comp. Example 7 | Comparative Example 8 |
|---|---|---|---|---|---|---|
| Drug Adhesive layer (wt%) | fentanyl | 8.0 | 6.67 | 8.0 | 8.0 | 6.67 |
| | nonfunctional polyacrylate adhesive | - | 75 | - | - | 84 |
| | Carboxyl-containing polyacrylate adhesive | 67 | 8.33 | 92 | 67 | 9.33 |
| | lauramine oxide | 25 | 10 | - | - | - |
| | Glyceryl monolaurate | - | - | - | 25 | - |
| | Sum | 100 | 100 | 100 | 100 | 100 |
| | drug adhesive layer thickness (*µ*m) | 50 | 63 | 50 | 60 | 60 |
| barrier layer thickness(*µ*m) | Rubber-based adhesive | 30 | 30 | 30 | 30 | 30 |
| Area per patch (cm²) | | 42 | 20 | 42 | 42 | 20 |
| Drug usage amount (mg/patch) | | 16.8 | 8.4 | 16.8 | 16.8 | 8.4 |
| Drug amount per unit area (mg/cm²) | | 0.4 | 0.42 | 0.4 | 0.4 | 0.4 |
| Drug permeation amount (mg/patch) | | 9.57 | 7.76 | 2.06 | 3.35 | 4.98 |
| Skin permeation rate (*µ*g/hr/cm²) | | 3.16 | 5.39 | 0.68 | 1.11 | 1.65 |
| Drug release amount per patch (%) | | 56.96 | 92.38 | 12.26 | 19.94 | 29.64 |

As shown in Table 2 and FIG. 4, the skin permeation rate of Example 2 using lauramine oxide as a skin permeation enhancer showed 1.5 to 1.6 times greater, compared to Comparative Examples 4 and 5 where the amounts of dipropylene glycol or glyceryl monolaurate used as the permeation enhancer of drug were high as twenty times as that of Example 2 and the drug concentration was higher than Example 2.

The skin permeation rate of Example 5 that the lauramine oxide and lauryl pyrrolidone were used together as skin permeation enhancer showed 2.2 times higher than those of Comparative Examples 4 and 5.

The trandermal preparation of Comparative Examples 6 using the carboxyl-containing polyacrylate adhesive showed a low skin permeation rate. However, when lauramine oxide was used in combination with the carboxyl-containing polyacrylate adhesive in Example 6, Example 6 showed the improved skin permeation rate effectively.

In the preparation of Example 7 using the mixture of nonfunctional polyacrylate adhesive and carboxyl-containing polyacrylate adhesive in drug adhesive layer at a suitable mixing ratio, it reduced the amount of additives and the property of preparation could be adjusted by changing the mixing ratio. Specifically, the used amount of lauramine oxide in Example 7 was 10 wt% which was less than 25 wt% in Example 6, Example 7 showed higher skin permeation rate.

### <Examples 8 to 10>

The trandermal preparations of Examples 8 and 9 were prepared according to the same method of Examples 3 and 5, respectively, except that Examples 8 and 9 used sufentanyl instead of fentanyl, and that the polyacrylate adhesive in the drug adhesive layer was acrylic acid ester copolymer TRM-05NF (Soken Chemical Co., Ltd., Japan) as a nonfunctional polyacrylate adhesive.

The trandermal preparation of Example 10 was prepared according to the same method of Example 7 where the drug adhesive layer contained the mixture of acrylic acid ester copoplymer TRM-05NF (Soken Chemical Co.,Ltd., Japan) as a nonfunctional polyacrylate adhesive, and acrylate/vinylacetate copolymer DURO-TAK 387-2052 (Henkel, U.S.A.) as a carboxyl-containing polyacrylate adhesive, except that sufentanyl was used instead of fentanyl and sufentanyl was 0.504 mg/cm² as a dry weight.

### <Comparative Examples 9 to 10>

The transdermal preparation of Comparative Example 9 was prepared according to the same method of Comparative Example 5, except that sufentanyl was used instead of fentanyl.

The transdermal preparation of Comparative Example 10 was prepared according to the same method of Comparative Example 8, except that sufentanyl was 0.504 mg/cm² as a dry weight.

### <Test Example 3>

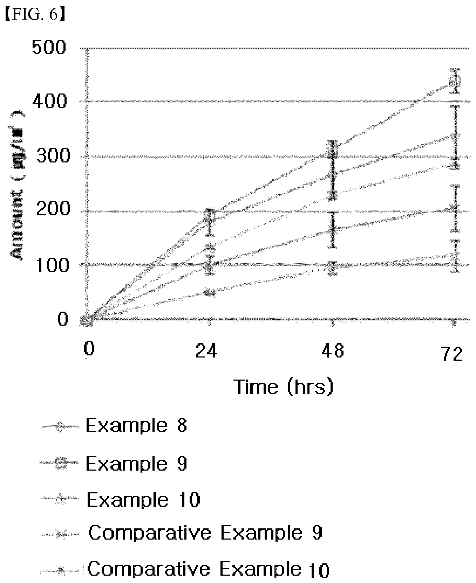

The transdermal preparations of Examples 8 to 10 and Comparative Examples 9 to 10, HPLC analyzing conditions were the same as those of Test Example 1, except for the changed conditions for sufentanyl. The test results were shown in Table 4 and FIG. 6.

### < HPLC Analysis Condition of sufentanyl>

Column: Capcell Pak C₁₈ UG120, 4.6 mm × 150 mm (Shiseido CO., LTD., Japan)
Mobile phase: 0.2% HClO₄ (pH 2.3 adjusted with NaOH): ACN = 55 : 45
UV:230 nm
Flow rate: 1.5 mℓ/minute
Sample injection amount: 10 *µ*ℓ

**[Table 4] Composition and properties of the preparations**

| Classfication | | Example 8 | Example 9 | Example 10 | Comparative Example 9 | Comparative Example 10 |
|---|---|---|---|---|---|---|
| Drug Adhesive layer (wt%) | sufentanyl | 6.67 | 5.7 | 6.67 | 10 | 6.67 |
| | nonfunctional polyacrylate adhesive | 92.83 | 88.7 | 75 | 90 | 84 |
| | Carboxyl-containing polyacrylate adhesive | - | - | 8.33 | - | 9.33 |
| | Lauramine oxide | 0.5 | 0.5 | 10 | - | - |
| | Lauryl pyrrolidone | - | 5.0 | - | - | - |
| | Sum | 100 | 100 | 100 | 100 | 100 |
| | drug adhesive layer thickness (*µ*m) | 75.6 | 88.4 | 75.6 | 50.4 | 75.6 |
| barrier layer thickness *µ*m) | Rubber-based adhesive | 30 | 30 | 30 | 30 | 30 |
| Area per patch (cm²) | | 20 | 20 | 20 | 20 | 20 |
| Drug usage amount (mg/patch) | | 10.08 | 10.08 | 10.08 | 10.08 | 10.08 |
| Drug amount per unit area (mg/cm²) | | 0.504 | 0.504 | 0.504 | 0.504 | 0.504 |
| Drug permeation amount (mg/patch) | | 6.79 | 8.81 | 5.74 | 4.12 | 2.37 |
| Skin permeation rate (*µ*g/hr/cm²) | | 4.717 | 6.117 | 3.985 | 2.863 | 1.646 |
| Drug release amount per patch (%) | | 67.39 | 87.38 | 56.93 | 40.9 | 23.51 |

As shown in Table 4 and FIG. 6, the skin permeation rate of Example 8 using lauramine oxide as a skin permeation enhancer showed 2.8 times greater, compared to Comparative Example 9 that did not contain the skin permeation enhancer in spite of a higher drug concentration.

Like the result of fentanyl, the preparing containing sufentanyl showed high permeation rate in Example 9 using the mixture of permeation enhancers.

## Claims

1. A transdermal preparation, comprising sequentially-stacked layers of a backing layer, a barrier layer, a drug adhesive layer and a release layer,
wherein the drug adhesive layer comprises
(a) a drug selected from the group consisting of fentanyl, an analogue and a pharmaceutically-acceptable salt thereof,
(b) a skin permeation enhancer which is at least one selected from the group consisting of lauramine oxide and N-dodecyl-2-pyrrolidone, and
(c) one or more polyacrylate adhesives selected from the group consisting of a non-functional polyacrylate adhesive and a carboxyl-containing polyacrylate adhesive,
wherein the drug is at least one selected from the group consisting of fentanyl, alfentanyl, sufentanyl, remifentanyl, 3-methylfentanyl, carfentanyl and a pharmaceutically acceptable salt thereof

2. The transdermal preparation according to Claim 1, wherein the drug is contained at an amount of 1 to 20 wt% based on the solid content of the drug adhesive layer.

3. The transdermal preparation according to Claim 1, wherein the polyacrylate adhesive of the drug adhesive layer is contained at an amount of 55 to 99 wt% based on the solid content of the drug adhesive layer.

4. The transdermal preparation according to Claim 1, wherein the polyacrylate adhesive of the drug adhesive layer is 80 to 99 wt% of the non-functional polyacrylate adhesive based on the solid content of the drug adhesive layer.

5. The transdermal preparation according to Claim 1, wherein the polyacrylate adhesive of the drug adhesive layer is 55 to 80 wt% of the carboxyl-containing polyacrylate adhesive based on the solid content of the drug adhesive layer.

6. The transdermal preparation according to Claim 1, wherein the polyacrylate adhesive of the drug adhesive layer is a mixture of the non-functional polyacrylate adhesive and Samyang Biopharmaceuticals Corporation the carboxyl-containing polyacrylate adhesive, at a mixing weight ratio of 95 : 5 to 55 : 45 for the non-functional polyacrylate: the carboxyl-containing polyacrylate.

7. The transdermal preparation according to Claim 1, wherein the nonfunctional polyacrylate adhesive is polymerized from at least one monomer selected from the group consisting of acrylic acid, 2-ethylhexylacrylate, hexylacrylate, butylacrylate, ethylacrylate, isooctylacrylate, vinylacetate, methylacrylate, methymethacrylate, ethylmethacrylate, acrylonitrile, hydroxyethylacrylate, octylacrylate, and t-octylacrylamide.

8. The transdermal preparation according to Claim 1, wherein the carboxyl-containing polyacrylate adhesive is polymerized from at least one monomer selected from the group consisting of acrylic acid, methaacrylic acid, crotonic acid, itaconic acid, fumaric acid and maleic acid, or
a copolymer polymerized from at least a monomer selected from the group consisting of acrylic acid, methaacrylic acid, crotonic acid, itaconic acid, fumaric acid, and maleic acid, and at least a monomer selected from the group consisting of acrylic acid, 2-ethylhexyl acrylate, hexyl acrylate, butyl acrylate, ethyl acrylate, isooctyl acrylate, vinyl acetate, methyl acrylate, methylmethacrylate, ethylmethacrylate, acrylonitrile, hydroxyethylacrylate, octylacrylate, and t-octylacrylamide.

9. The transdermal preparation according to Claim 1, wherein the skin permeation enhancer is contained at an amount of 0.1 to 50 wt% based on the solid content of the drug adhesive layer.

10. The transdermal preparation according to Claim 1, wherein the barrier layer comprises at least a rubber-based adhesive selected from the group consisting of polyisobutylene (PIB), polyisoprene, polybutadiene and polybutene.

11. The transdermal preparation according to Claim 10, wherein the barrier layer comprises at least an additive selected from the group consisting of a tackifying resin of C₃₋₁₂ aliphatic hydrocarbon resin and a plasticizer.

12. The transdermal preparation according to Claim 1, wherein the barrier layer has a dry layer thickness of 10 to 60 µm.

13. A transdermal preparation according to Claim 1, having 1.05 to 10.08 mg of the drug content per unit dosage, 3.0 to 6.5 µm/hr/cm² of the drug permeation rate, and 50 to 95 wt% of drug releasing amount per unit dosage to the initial loading amount of drug.

## Patentansprüche

1. Transdermales Präparat, welches aufeinanderfolgend gestapelte Schichten einer Trägerschicht, einer Sperrschicht, einer Arzneimittelklebeschicht und einer Freigabeschicht aufweist,
wobei die Arzneimittelklebeschicht aufweist:
(a) ein Arzneimittel, das aus der Gruppe ausgewählt worden ist, welche ein Fentanyl, ein Analogon und ein pharmazeutisch verträgliches Salz davon umfasst,
(b) ein Verstärkungsmittel der Hautdurchdringung, das mindestens eines ist, das aus der Gruppe ausgewählt worden ist, welche ein Laurinooxid und ein N-Dodecyl-2-Pyrrolidon umfasst, und
(c) einen oder mehrere Polyacrylat-Klebstoffe, die aus der Gruppe ausgewählt worden sind, welche einen nicht-funktionellen Polyacrylat-Klebstoff und einem carboxylhaltigen Polyacrylat-Klebstoff umfasst,
wobei das Arzneimittel mindestens eines ist, das aus der Gruppe ausgewählt worden ist, welche ein Fentanyl, ein Alfentanyl, ein Sufentanyl, ein Remifentanyl, ein 3-Methylfentanyl, ein Carfentanyl und ein pharmazeutisch verträgliches Salz umfasst.

2. Transdermales Präparat nach Anspruch 1, wobei das Arzneimittel in einer Menge von 1 bis 20 Gewichtsprozent bezogen auf den Feststoffgehalt der Arzneimittelklebeschicht enthalten ist.

3. Transdermales Präparat nach Anspruch 1, wobei der Polyacrylat-Klebstoff der Arzneimittelkleberschicht in einer Menge von 55 bis 99 Gewichtsprozent bezogen auf den Feststoffgehalt der Arzneimittelklebeschicht enthalten ist.

4. Transdermales Präparat nach Anspruch 1, wobei der Polyacrylat-Klebstoff der Arzneimittelklebeschicht in einer Menge von 80 bis 99 Gewichtsprozent des nichtfunktionellen Polyacrylat-Klebstoffes bezogen auf den Feststoffgehalt der Arzneimittelklebeschicht enthalten ist.

5. Transdermales Präparat nach Anspruch 1, wobei der Polyacrylat-Klebstoff der Arzneimittelklebeschicht in einer Menge von 55 bis 80 Gewichtsprozent des carboxylhaltigen Polyacrylat-Klebstoff bezogen auf den Feststoffgehalt der Arzneimittelklebeschicht enthalten ist.

6. Transdermales Präparat nach Anspruch 1, wobei der Polyacrylat-Klebstoff der Arzneimittelklebeschicht eine Mischung aus dem nicht-funktionellen Polyacrylat-Klebstoff und dem carboxylhaltigen Polyacrylat-Klebstoff in einem Mischungsgewichtsverhältnis von 95 zu 5 bis 55 zu 45 für das nicht-funktionelle Polyacrylat zu dem carboxylhaltigen Polyacrylat ist.

7. Transdermales Präparat nach Anspruch 1, wobei der nichtfunktionelle Polyacrylat-Klebstoff aus mindestens einem Monomer polymerisiert worden ist, das aus der Gruppe ausgewählt worden ist, welche eine Acrylsäure, ein 2-Ethylhexylacrylat, ein Hexylacrylat, ein Butylacrylat, ein Ethylacrylat, ein Isooctylacrylat, ein Vinylacetat, ein Methylacrylat, ein Methymethacrylat, ein Ethylmethacrylat, ein Acrylnitril, ein Hydroxyethylacrylat, ein Octylacrylat und ein t-Octylacrylamid umfasst.

8. Transdermales Präparat nach Anspruch 1, wobei der carboxylhaltige Polyacrylat-Klebstoff aus mindestens einem Monomer polymerisiert worden ist, das aus der Gruppe ausgewählt worden ist, welche eine Acrylsäure, eine Methacrylsäure, eine Crotonsäure, eine Itaconsäure, eine Fumarsäure und eine Maleinsäure umfasst, oder
ein Copolymer ist, das aus mindestens einem Monomer, das aus der Gruppe ausgewählt worden ist, welche eine Acrylsäure, eine Methacrylsäure, eine Crotonsäure, eine Itaconsäure, eine Fumarsäure und eine Maleinsäure umfasst, und aus mindestens einem Monomer polymerisiert worden ist, das aus der Gruppe ausgewählt worden ist, welche eine Acrylsäure, ein 2-Ethylhexyl Acrylat, ein Hexylacrylat, ein Butylacrylat, ein Ethylacrylat, ein Isooctylacrylat, ein Vinylacetat, ein Methylacrylat, ein Methylmethacrylat, ein Ethylmethacrylat, ein Acrylnitril, ein Hydroxyethylacrylat, ein Octylacrylat und ein t-Octylacrylamid umfasst.

9. Transdermales Präparat nach Anspruch 1, wobei das Verstärkungsmittel der Hautdurchdringung in einer Menge von 0,1 bis 50 Gewichtsprozent bezogen auf den Feststoffgehalt der Arzneimittelklebeschicht enthalten ist.

10. Transdermales Präparat nach Anspruch 1, wobei die Sperrschicht mindestens einen Klebstoff auf Kautschukbasis aufweist, der aus der Gruppe ausgewählt worden ist, welche ein Polyisobutylen (PIB), ein Polyisopren, ein Polybutadien und ein Polybuten umfasst.

11. Transdermales Präparat nach Anspruch 10, wobei die Sperrschicht mindestens ein Additiv aufweist, das aus der Gruppe ausgewählt worden ist, welche ein klebrig machendes Harz aus einem aliphatischem C3-12-Kohlenwasserstoffharz und einem Weichmacher umfasst.

12. Transdermales Präparat nach Anspruch 1, wobei die Sperrschicht eine Trockenschichtdicke von 10 bis 60 µm aufweist.

13. Transdermales Präparat nach Anspruch 1, welche 1,05 bis 10,08 mg des Arzneimittelgehalts pro Einheitsdosierung, 3,0 bis 6,5 µm/h/cm² der Arzneimittelpermeationsrate und 50 bis 95 Gewichtsprozent der Arzneimittelfreigabemenge pro Einheitsdosierung zu der anfängliche Beladungsmenge des Arzneimittels aufweist.

## Revendications

1. Préparation transdermique, comprenant des couches séquentiellement empilées d'une couche de support, d'une couche barrière, d'une couche adhésive de médicament et d'une couche anti-adhésive,
dans laquelle la couche adhésive de médicament comprend
(a) un médicament sélectionné dans le groupe constitué du fentanyl, d'un analogue et d'un sel pharmaceutiquement acceptable de celui-ci,
(b) un agent augmentant la pénétration à travers la peau qui est au moins un agent sélectionné dans le groupe constitué de l'oxyde de lauramine et de la N-dodécyl-2-pyrrolidone, et
(c) un ou plusieurs adhésifs à base de polyacrylate sélectionnés dans le groupe constitué d'un adhésif à base de polyacrylate non fonctionnel et d'un adhésif à base de polyacrylate contenant un groupe carboxyle,
dans laquelle le médicament est au moins un sélectionné dans le groupe constitué du fentanyl, de l'alfentanyl, du sufentanyl, du rémifentanyl, du 3-méthylfentanyl, du carfentanyl et d'un sel pharmaceutiquement acceptable de ceux-ci.

2. Préparation transdermique selon la revendication 1, dans laquelle le médicament est contenu en une quantité de 1 à 20 % en poids sur la base de la teneur en matières solides de la couche adhésive de médicament.

3. Préparation transdermique selon la revendication 1, dans laquelle l'adhésif à base de polyacrylate de la couche adhésive de médicament est contenu en une quantité de 55 à 99 % en poids sur la base de la teneur en matières solides de la couche adhésive de médicament.

4. Préparation transdermique selon la revendication 1, dans laquelle l'adhésif à base de polyacrylate de la couche adhésive de médicament représente 80 à 99 % en poids de l'adhésif à base de polyacrylate non fonctionnel sur la base de la teneur en matières solides de la couche adhésive de médicament.

5. Préparation transdermique selon la revendication 1, dans laquelle l'adhésif à base de polyacrylate de la couche adhésive de médicament représente 55 à 80 % en poids de l'adhésif à base de polyacrylate contenant un groupe carboxyle sur la base de la teneur en matières solides de la couche adhésive de médicament.

6. Préparation transdermique selon la revendication 1, dans laquelle l'adhésif à base de polyacrylate de la couche adhésive de médicament est un mélange de l'adhésif à base de polyacrylate non fonctionnel et de l'adhésif à base de polyacrylate contenant un groupe carboxyle, en un rapport pondéral de mélange de 95/5 à 55/45 pour le polyacrylate non fonctionnel/le polyacrylate contenant un groupe carboxyle.

7. Préparation transdermique selon la revendication 1, dans laquelle l'adhésif à base de polyacrylate non fonctionnel est polymérisé à partir d'au moins un monomère sélectionné dans le groupe constitué de l'acide acrylique, de l'acrylate de 2-éthylhexyle, de l'acrylate d'hexyle, de l'acrylate de butyle, de l'acrylate d'éthyle, de l'acrylate d'isooctyle, de l'acétate de vinyle, de l'acrylate de méthyle, du méthacrylate de méthyle, du méthacrylate d'éthyle, de l'acrylonitrile, de l'acrylate d'hydroxyéthyle, de l'acrylate d'octyle, et de l'acrylamide de t-octyle.

8. Préparation transdermique selon la revendication 1, dans laquelle l'adhésif à base de polyacrylate contenant un groupe carboxyle est polymérisé à partir d'au moins un monomère sélectionné dans le groupe constitué de l'acide acrylique, de l'acide méthacrylique, de l'acide crotonique, de l'acide itaconique, de l'acide fumarique et de l'acide maléique, ou
d'un copolymère polymérisé à partir d'au moins un monomère sélectionné dans le groupe constitué de l'acide acrylique, de l'acide méthacrylique, de l'acide crotonique, de l'acide itaconique, de l'acide fumarique, et de l'acide maléique, et d'au moins un monomère sélectionné dans le groupe constitué de l'acide acrylique, de l'acrylate de 2-éthylhexyle, de l'acrylate d'hexyle, de l'acrylate de butyle, de l'acrylate d'éthyle, de l'acrylate d'isooctyle, de l'acétate de vinyle, de l'acrylate de méthyle, du méthacrylate de méthyle, du méthacrylate d'éthyle, de l'acrylonitrile, de l'acrylate d'hydroxyéthyle, de l'acrylate d'octyle, et de l'acrylamide de t-octyle.

9. Préparation transdermique selon la revendication 1, dans laquelle l'agent augmentant la pénétration à travers la peau est contenu en une quantité de 0,1 à 50 % en poids sur la base de la teneur en matières solides de la couche adhésive de médicament.

10. Préparation transdermique selon la revendication 1, dans laquelle la couche barrière comprend au moins un adhésif à base de caoutchouc sélectionné dans le groupe constitué du polyisobutylène (PIB), du polyisoprène, du polybutadiène et du polybutène.

11. Préparation transdermique selon la revendication 10, dans laquelle la couche barrière comprend au moins un additif sélectionné dans le groupe constitué d'une résine collante d'une résine d'hydrocarbure aliphatique en C₃₋₁₂ et d'un plastifiant.

12. Préparation transdermique selon la revendication 1, dans laquelle la couche barrière a une épaisseur de couche sèche de 10 à 60 µm.

13. Préparation transdermique selon la revendication 1, ayant une teneur en médicament de 1,05 à 10,08 mg par dose unitaire, un taux de pénétration du médicament de 3,0 à 6,5 µm/h/cm², et une quantité de libération de médicament de 50 à 95 % en poids par dose unitaire par rapport à la quantité de charge de médicament initiale.
